# FASCICULE DE BREVET EUROPEEN

(11) **EP 0 614 910 B1**
(45) Date de publication et mention de la délivrance du brevet: **18.11.1998**
(21) Numéro de dépôt: 94400326.8
(22) Date de dépôt: 15.02.1994
(51) Int. Cl.: A61K 38/05, A61K 38/06

(54) **Forme purifiée de streptogramines, sa préparation et les compositions pharmaceutiques qui la contiennent**
Streptogramine in gereinigte Form, ihre Herstellung und sie enthaltende pharmazeutische Zusammensetzungen
Streptogramines in purified form, their preparation and pharmaceutical compositions comprising them

(30) Priorité: 17.02.1993 FR 9301787
(43) Date de publication de la demande: 14.09.1994
(73) Titulaire: RHONE-POULENC RORER S.A., 92160 Antony (FR)
(72) Inventeur: Anger, Pascal, F-91370 Verrières-le-Buisson (FR); Bonnavaud, Bertrand, F-78220 Viroflay (FR); Callet, Alain, F-94310 Orly (FR); Lefevre, Patrick, F-94300 Vincennes (FR)
(74) Mandataire: Savina, Jacques

(56) Documents cités:
- EP-A- 0 506 561
- WO-A-93/20182
- FR-A- 2 619 008
- BULLETIN DE LA SOCIETE CHIMIQUE DE FRANCE vol. 2 , 1968 , PARIS FR pages 585 - 591 J. PREUD'HOMME ET AL. 'Pristinamycine isolement, caractérisation et identification des constituants'
- CHEMICAL ABSTRACTS, vol. 110, no. 7, 13 Février 1989, Columbus, Ohio, US; abstract no. 56082, N.K. SHARMA ET AL. 'Isolation of factor A (virginamycin M1) and factor B (mixture of VS1 and VS4) from a commercial fee additive formulation' page 551 ;colonne R ; & BULLETIN DES SOCIETES CHIMIQUES BELGES vol. 97, no. 3 , 1988 , OXFORD GB pages 185 - 192

## Description

La présente invention concerne une forme purifiée de streptogramines comprenant au moins une composante du groupe B des streptogramines associée à au moins une composante "minoritaire" du groupe A définie ci-après par la formule générale (II).

Parmi les streptogramines connues, la pristinamycine (RP 7293), anti-bactérien d'origine naturelle produit par *Streptomyces pristinaespiralis* a été isolée pour la première fois en 1955. La pristinamycine commercialisée sous le nom de Pyostacine® est constituée principalement de pristinamycine IA et de pristinamycine IIA.

Un autre antibactérien de la classe des streptogramines : la virginiamycine, a été préparé à partir de *Streptomyces virginiae,* ATCC 13161 [Antibiotics and Chemotherapy, 5, 632 (1955)]. La virginiamycine (Staphylomycine®) est constituée principalement de facteur S et de facteur M₁.

Dans le brevet US 3 325 359 ont été décrites des compositions pharmaceutiques comprenant des substances antibiotiques constituant l'antibiotique 899 : facteur S et facteur M1.

Dans la demande de brevet FR 2 619 008 a été décrite l'utilisation de composantes du groupe A et du groupe B pour le traitement de l'acnée.

Les antibactériens d'origine naturelle, de la classe des streptogramines sont constitués du mélange de 2 groupes de composants composantes du groupe B et composantes du groupe A, chaque groupe ayant une activité antibactérienne propre. Il a été démontré que l'association formée par les 2 groupes des composantes provoque une synergie d'action qui résulte dans une activité bactériostatique et bactéricide accrue et dans l'élargissement du spectre d'activité.

Dans Streptogramine als Modelsysteme für den Kationentransport durch Membranen, Dissertation zur Erlangung des Doktorgrades der Mathematisch-Naturwissenschaftlichen Facultät der Georg-August Universität zu Göttingen, Göttingen 1979, dans Antibiotics III, 521 (1975) et dans Antibiotics of the virginiamycin family, Inhibitors which contain synergistic components, C. Cocito, Microbiological Reviews, 145-98 (1979) ont été décrites des composantes des groupes A et B des streptogramines. J. Preud'Homme, P. Tarridec, et A. Belloc, Bull. Soc. Chim. Fr., 2, 585 (1968) ont également décrit la pristinamycine naturelle ainsi que les différentes composantes qui la constituent.

Toutes les tentatives d'associations purifiées de streptogramines font systématiquement intervenir la composante majoritaire du groupe A [pristinamycine IIA (PIIA)] considérée comme responsable de l'activité et de la synergie d'action. Des études ont d'ailleurs conclu à l'importance de cette composante qui provoque une meilleure synergie : EP 506 561 (page 2).

Cependant, ces tentatives n'ont jamais été couronnées de succès, d'une part à cause des difficultés de préparation industrielle et surtout parce que la pristinamycine IIA purifiée est un produit cristallisé dont la biodisponibilité s'est avérée trop faible pour pouvoir envisager d'en faire le principe actif d'un médicament.

Du point de vue de la préparation industrielle de tels produits, jusqu'à présent, les techniques à disposition n'avaient pas permis d'obtenir, à une échelle préparative, une forme suffisamment purifiée et la production de lots de qualité suffisamment constante et reproductible pour répondre aux exigences des législations de certains pays en matière d'enregistrement.

A titre d'exemple, les lots industriels de pristinamycine naturelle contiennent après purification, une quantité d'impuretés pouvant atteindre 20 %. Les tentatives de purification mises en oeuvre jusqu'à présent ont systématiquement abouti à des échecs et bien souvent à la dégradation de l'un des groupes de composants du fait qu'il s'agit de produits fragiles pour lesquels de nombreuses opérations aboutissent à l'ouverture de la structure cyclisée ou à la deshydratation des composantes du groupe A. De ce fait, pendant de nombreuses années il a été considéré qu'une amélioration du degré de pureté ne pouvait pas être atteinte. En 1988, la purification était encore considérée comme un problème : J. of Liq. Chromatography, 11(11), 2367 (1988). Egalement en 1988, N.K. SHARMA et M.J.O. ANTEUNIS déclaraient aussi que la séparation et la purification des composantes de la virginiamycine était possible à des fins analytiques mais pas envisageable pour la production des produits, compte tenu des difficultés rencontrées : Bull. Soc. Chim. Belg., 97(3) 193 (1988).

En conséquence de cette situation, la commercialisation de la pristinamycine (Pyostacine®) a été définitivement limitée à certains pays comme la France et la Belgique. Il en a été de même pour la virginiamycine (Staphylomycine®) commercialisée seulement dans un nombre limité de pays en ce qui concerne la médecine humaine, ainsi que pour la mikamycine dont la commercialisation (limitée au Japon) est maintenant arrêtée. Ceci a donc abouti pour certaines populations, à la privation d'un traitement dans le cas d'infections graves à cocci gram positif (notamment les infections à staphylococci résistant à la méthicilline) ou d'un traitement dans le cas de maladies sexuellement transmissibles.

Dans le domaine des antibactériens, il est bien connu des praticiens que des allergies ou des résistances peuvent se développer après administration de certaines classes d'antibiotiques [The New England Journal of Medicine, 324 (9), 601 (1991)]. En milieu hospitalier on connaît notamment de nombreuses souches résistantes de Staphylococcus aureus. C'est pourquoi il est extrêmement utile pour le médecin d'avoir à sa disposition un large éventail de classes chimiquement différentes de manière à pouvoir adapter le traitement au cas particulier du malade à traiter. La conséquence de l'absence de commercialisation d'une classe déterminée peut être très grave, voire dramatique puisqu'elle peut résulter dans la privation de traitement chez des malades ne tolérant pas les autres classes d'antibiotiques.

Ainsi, les essais de purification mis en oeuvre avaient toujours eu pour but d'éliminer les composantes minoritaires des streptogramines, celles-ci étant considérées comme non indispensables et plutôt comme des impuretés.

Parmi les composantes du groupe A des streptogramines naturelles, la pristinamycine IIB (PIIB) est une composante minoritaire dont la proportion en poids est inférieure à 10 % dans la pristinamycine naturelle, et le plus souvent de l'ordre de 8 % ou même de l'ordre de 6 % dans la virginiamycine.

Il a été trouvé maintenant, et c'est ce qui fait l'objet de la présente invention, que l'association constituée d'une ou plusieurs composantes du groupe B, de formule générale : 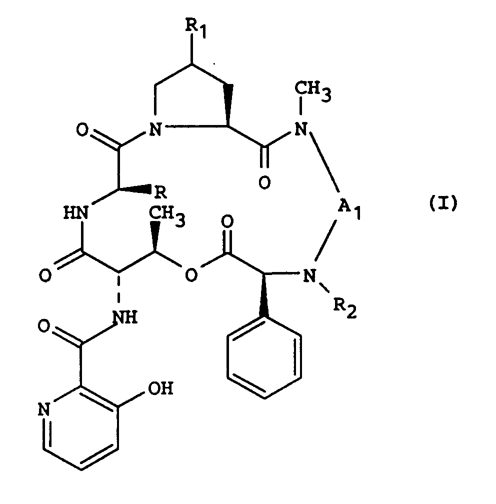
dans laquelle A₁ est un radical de formule générale : 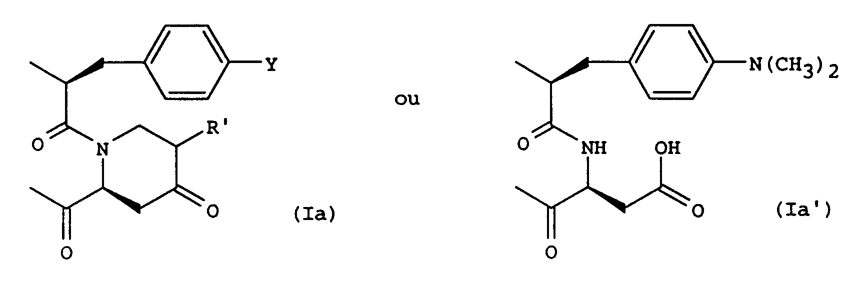
pour lequel R' est un atome d'hydrogène ou un radical hydroxy et Y est un atome d'hydrogène, un radical méthylamino ou un radical diméthylamino,
R est un radical éthyle ou lorsque R' représente un atome d'hydrogène
R peut également représenter un radical méthyle, et
R₁ et R₂ représentent un atome d'hydrogène, ou bien
A₁ est un radical de formule : 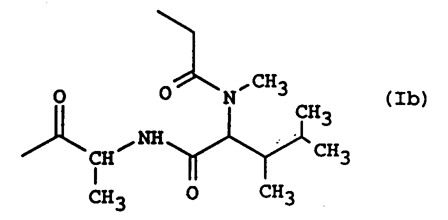
R est un radical isobutyle, et
R₁ est un radical hydroxy et R₂ est un radical méthyle, et d'une ou plusieurs composantes "minoritaires" du groupe A, de formule générale : 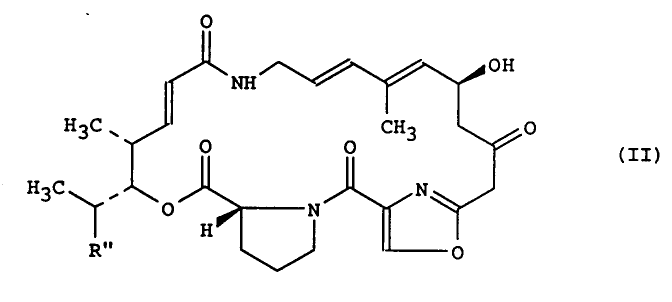
dans laquelle R'' est un atome d'hydrogène ou un radical méthyle ou éthyle, est particulièrement intéressante du fait de son activité biologique in vivo.

En effet les associations selon l'invention manifestent une action biologique in vivo nettement supérieure à celle du produit naturel (par exemple la virginiamycine naturelle ou la pristinamycine naturelle) ou à celle des associations faisant intervenir la composante majoritaire du groupe A et surtout sont doués d'une biodisponibilité tout à fait satisfaisante. De plus ces associations peuvent être préparées à une échelle importante.

Il est ainsi possible d'accéder à une forme purifiée et biodisponible d'un produit final de bon niveau d'activité, contenant moins de 6 % d'impuretés.

Le produit de formule générale (II) pour lequel R'' est un radical éthyle ci-après appelé pristinamycine IIF (PIIF) et le produit de formule générale (II) pour lequel R'' est un atome d'hydrogène ci-après appelé pristinamycine IIG (PIIG) sont des produits nouveaux qui constituent des composantes très minoritaires des streptogramines dont la proportion en poids est inférieure à 0,5 % dans les lots de produit naturel.

Les associations selon l'invention sont avantageusement préparées dans des proportions de 10/90 à 90/10 (en poids) ou de préférence dans les proportions de 20/80 à 80/20. Elles se présentent à l'état de mélange physique des poudres, mais aussi, ce qui constitue un autre aspect de la présente invention, à l'état de coprécipité ; ou encore selon un troisième aspect de l'invention, à l'état de cocristallisat tel que défini ci-après.

La présente invention concerne aussi les formes purifiées constituées par l'association cocristallisée d'au moins une composante du groupe B de formule générale (I) avec au moins une composante du groupe A définie par la formule générale (II).

La cocristallisation s'effectue dans la stoechiométrie constante de 1 mole de composante(s) de formule générale (I) avec 2 moles de composante(s) du groupe A de formule générale (II) [cette stoechiométrie correspondant à la proportion relative d'environ 43-44/57-56 en poids dans le cas où la composante A est un produit de formule générale (I) pour lequel A₁ est de structure (Ia)].

L'association cocristallisée selon l'invention peut être utilisée alternativement comme agent antimicrobien purifié et stable, et possédant aussi une activité in vivo améliorée ainsi qu'une bonne biodisponibilité, ou bien comme moyen de purification d'une composante minoritaire des streptogramines répondant à la formule générale (II).

En effet, il n'a jamais été possible de purifier une composante du groupe A de formule générale (II) par cristallisation ; de ce fait, jusqu'à présent, seules des méthodes chromatographiques étaient connues pour préparer un produit purifié du groupe A de formule générale (II), et aucun autre moyen de purification n'était connu pour permettre d'isoler ces produits en quantités importantes.

Il a maintenant été montré que la composante du groupe A de formule générale (II) peut être obtenue à l'état pur en passant par l'intermédiaire de l'association cocristallisée définie ci-dessus. Un mélange brut contenant au moins 30 % d'une composante minoritaire du groupe A répondant à la formule générale (II) mis en solution dans un solvant organique tel qu'une cétone (acétone, méthyléthylcétone, méthylisobutylcétone par exemple), un ester (acétate d'éthyle, acétate d'isopropyle, acétate de butyle, acétate d'isobutyle par exemple), un solvant chloré (chlorure de méthylène, chloroforme, dichloro-1,2 éthane par exemple), ou un nitrile (acétonitrile par exemple), et additionné d'une composante du groupe B définie par la formule générale (I) donne un composé cocristallisé dans les proportions définies ci-avant. Il est entendu que la quantité de composé de formule générale (I) introduite est choisie convenablement pour que la concentration résiduelle de ce produit (après la cocristallisation) soit inférieure à sa solubilité dans le milieu. Il est également entendu que des variations dans les teneurs respectives du milieu initial en produit de formule générale (II) et en produit de formule générale (I) n'entrainent pas de modification du composé cocristallisé obtenu. L'association cocristallisée ainsi obtenue, mise en solution dans un solvant comme par exemple la méthylisobutylcétone ou le dichloroéthane et traitée en milieu acide (acide sulfurique, acide chlorhydrique par exemple) permet d'obtenir après traitement de la phase organique par un solvant comme par exemple l'hexane, la composante minoritaire du groupe A, purifiée et exempte de composante du groupe B.

Cette association cocristallisée présente par ailleurs l'avantage d'une stabilité fortement accrue, d'une pureté élevée et surtout de permettre une industrialisation aisée.

Il est bien entendu que cette méthode peut être aussi adaptée à la préparation de cocristallisats avec des dérivés modifiés des composantes naturelles du groupe B des streptogramines et que ces cocristallisats entrent aussi dans le cadre de la présente invention ; de même la préparation des formes purifiées de composantes minoritaires de formule générale (II) à partir de tels cocristallisats entre également dans le cadre de la présente invention.

Un mode préféré de réalisation de l'invention concerne une association de pristinamycine IB [telle que définie ci-dessus lorsque A₁ représente un radical de formule générale (Ia) pour lequel Y est un radical méthylamino et R' est un atome d'hydrogène, et R est un radical éthyle] ou de virginiamycine S1 [telle que définie ci-dessus lorsque A₁ représente un radical de formule générale (Ia) pour lequel Y et R' sont des atomes d'hydrogène, et R est un radical éthyle] ou d'un mélange de virginiamycine S1 et de virginiamycine S4 [telle que définie ci-dessus lorsque A₁ est défini comme pour la virginiamycine S1 et R est un radical méthyle] avec la pristinamycine IIB [telle que définie ci-dessus par la formule générale (II) dans laquelle R'' est méthyle] et contenant moins de 6 % d'impuretés et de préférence moins de 3 % d'impuretés.

Un mode préféré de réalisation de l'invention concerne également une streptogramine purifiée constituée de l'association d'une composante du groupe B des streptogramines définie par la formule générale (I) et d'une composante du groupe A de formule générale (II) contenant une proportion relative des composantes des groupes B et A dans un rapport molaire constant de l'ordre de 1/2.

Selon l'invention, les nouvelles associations d'au moins une composante du groupe B des streptogramines de formule générale (I) et d'au moins une composante du groupe A de formule générale (II) peuvent être obtenues par exemple par préparation du composé cocristallisé tel que défini ci-dessus. Lorsque l'on veut obtenir une association dans des proportions différentes, le composé cocristallisé ainsi préparé peut être associé avec au moins une des composantes de formule générale (I) ou avec au moins une composante du groupe A de formule générale (II) préalablement purifiées et en quantité convenable pour obtenir les proportions souhaitées, ou bien la composante du groupe A de formule générale (II) (ou leur mélange) peut être purifiée à partir du cocristallisat puis mélangée dans les proportions souhaitées avec une ou plusieurs composantes du groupe B de formule générale (I). Alternativement les associations selon l'invention peuvent être préparées après isolement de la(des) composante(s) du groupe B et de la composante du groupe A de formule générale (II) à partir de la streptogramine naturelle correspondante, par purification de chacune de ces composantes, puis mélange des composantes purifiées, dans les proportions souhaitées telles que définies précédemment.

Les associations selon l'invention peuvent également être coprécipitées dans les proportions souhaitées, à partir d'une solution des composantes de formule générale (I) et (II) [ou alternativement d'une solution du cocristallisat et de l'une des composantes de formule générale (I) ou (II)] dans la méthylisobutylcétone, dans l'acétone ou le chlorure de méthylène, versée dans l'hexane, le cyclohexane ou dans l'eau.

La préparation et la séparation des composantes des groupes A et B est effectuée par fermentation et isolement des constituants à partir du môut de fermentation selon ou par analogie avec la méthode décrite par J. Preud'homme et coll., Bull. Soc. Chim. Fr., vol.2, 585 (1968), dans Antibiot. & Chemother., 5, 632 (1955) ou 7, 606 (1957), dans Chromatog. Syn., 2° Bruxelles, 181 (1962), dans Antibiot. Ann., 728 784 (1954-55), dans le brevet US 3 299 047 ou dans Streptogramine als Modelsysteme für den Kationentransport durch Membranen, Dissertation zur Erlangung des Doktorgrades der Mathematisch-Naturwissenschaftlichen Facultät der Georg-August Universität zu Göttingen, Göttingen 1979 ou comme décrit ci-après dans les exemples. Notamment dans le cas des pristinamycines, la séparation des composantes des groupes A et B est effectuée par mise en suspension de la streptogramine brute dans un solvant organique comme un acétate (acétate d'éthyle par exemple) suivie de la filtration ou de la centrifugation de la composante brute du groupe A, et de l'extraction de la composante du groupe B en milieu acide aqueux suivie d'une réextraction en milieu chlorométhylènique. La séparation des composantes des groupes A et B peut également être effectuée par extraction acide d'une solution de streptogramine brute dans la méthylisobutylcétone, puis isolement par extraction de la composante du groupe B à partir de la phase aqueuse et isolement de la composante du groupe A par précipitation à partir de la phase organique.

Après séparation, la purification des composantes du groupe B des streptogramines peut être mise en oeuvre par cristallisation dans un alcool tel que l'éthanol, le méthanol ou l'isopropanol, dans un acétate (acétate d'isopropyle ou acétate de butyle par exemple), dans une cétone (méthyléthylcétone par exemple) ou dans l'acétonitrile ou par chromatographie. La purification des composantes du groupe A de formule générale (II) peut être effectuée par chromatographie en éluant par un mélange acétonitrile-eau.

Alternativement la préparation des composantes des groupes A et B respectivement de formule générale (II) et (I) est effectuée comme décrit dans la demande de brevet français 2 689 518, par fermentation séparée selon les étapes suivantes :
- première étape (facultative), mutagénèse sur un microorganisme producteur de streptogramines non-sélectif, et,
- deuxième étape, sélection des microorganismes sélectifs.

Les microorganismes non-sélectifs sont généralement des Actinomycètes et des champignons. Les microorganismes de départ utilisables dans le procédé sont notamment des microorganismes producteurs non-sélectifs d'une streptogramine choisie parmi le groupe comprenant la pristinamycine, la virginiamycine, la mikamycine, l'ostréogrycine, la viridogriséine, la vernamycine et l'étamycine. A titre d'exemple, des microorganismes non-sélectifs pouvant être employés sont cités ci-après dans le tableau.

| **MICROORGANISMES** | **ANTIBIOTIQUES** |
|---|---|
| ***CHAMPIGNONS*** | |
| Micromonospora sp. | vernamycine |

| ***STREPTOMYCES*** | |
|---|---|
| S.alborectus | virginiamycine |
| S.griseus (NRRL2426) | viridogriséine |
| S.lavendulae | étamycine |
| S.loïdensis (ATCC11415) | vernamycine |
| S.mitakaensis (ATCC15297) | mikamycine |
| S.ostreogriseus (ATCC27455) | ostréogrycine |
| S.pristinaespiralis (ATCC25486) | pristinamycine |
| S.virginiae (ATCC13161) | virginiamycine |

| ***ACTINOMYCES*** | |
|---|---|
| A.daghestanicus | etamycine |

Plus particulièrement, la préparation est mise en oeuvre à partir de microorganismes choisis parmi Streptomyces alborectus, Streptomyces mitakaensis, Streptomyces pristinaespiralis, Streptomyces ostreogriséus et Streptomyces virginiae.

La première étape de la préparation consiste à modifier le microorganisme non-sélectif, de manière à augmenter ses capacités globales de production d'antibiotique, et/ou à ce qu'il ne synthétise qu'une seule des 2 composantes des streptogramines. Ceci peut être obtenu par modifications génétiques (mutation au niveau de gènes de structure d'enzymes impliquées dans la voie de biosynthèse, ou au niveau des séquences permettant l'expression de tels gènes de structure par exemple) ou biochimiques (modification d'un mécanisme post-traductionnel, altération d'un mécanisme de rétroinhibition etc..). Différents outils de mutagénèse sont utilisés :
- des agents physiques : rayons X, rayons Ultra Violet ; ou,
- des agents chimiques : des agents alkylants tels que l'éthylméthanesulfonate (EMS), la N-méthyl-N'-nitro-N-nitrosoguanidine (Delic et al. Mutation Res. 9 (1970) 167-182), ou la 4-nitroquinoléine-1-oxyde (NQO) ; des agents bialkylants ; des agents intercalants ; ou,
- tout système d'insertion mutationnel sur l'ADN, et en particulier les transposons, les plasmides intégratifs, les phages ou les prophages ; ou encore,
- la fusion de protoplastes (Cohen, Nature 268 (1977) 171-174).

Ces outils (seuls ou en combinaison) peuvent être appliqués sur les microorganismes non-sélectifs à l'état de spores, de spores germées ou en cours de germination ou sur mycélium. La préparation peut aussi faire appel à des manipulations (au hasard ou dirigées) permettant d'obtenir des microorganismes capables de produire sélectivement une composante des streptogramines à partir de microorganismes non-sélectifs.

La seconde étape de la préparation concerne l'identification et l'isolement des microorganismes sélectifs. Cette étape peut être réalisée notamment au moyen d'un test de sensibilité vis-à-vis d'un germe. Différents germes sensibles spécifiquement aux composantes du groupe A ou à celles du groupe B des streptogramines existent : par exemple Bacillus subtilis (ATCC6633), Bacillus circulans, Bacillus cereus (Watanabe, J. Antibio. Ser. A XIII(1) (1960) 62), ou C.xerosis (Watanabe précitée) qui sont sensibles spécifiquement aux composantes du groupe B; Streptococcus agalactiae B96 (Antimicrob. Agents Chemother. 10(5) (1976) 795), Micrococcus luteus (Prikrylova précitée) ou Sarcina lutea (ATCC9341) qui sont sensibles spécifiquement aux composantes du groupe A. Il est aussi possible de préparer artificiellement des germes sensibles spécifiquement à une composante des streptogramines en insérant, dans un germe sensible aux 2 composantes des streptogramines, un gène de résistance à l'une d'entre-elles. Certains de ces gènes ont été clonés (Le Goffic et al., J. Antibio. XXX(8), 665 (1977) ; Le Goffic et al., Ann. Microbiol. Inst, Pasteur 128B, 471 (1977) ; Solh et al., Path. Biol. 32(5), 362, (1984), de tels gènes sont introduits dans différents germes par des techniques classiques de biologie moléculaire. L'étape de sélection peut également être effectuée par test ELISA au moyen d'anticorps spécifiques des composantes A ou B, ou encore par des techniques analytiques telles que la chromatographie (chromatographie liquide, chromatographie sur couche mince, etc..). Dans le cas d'un test de sensibilité vis-à-vis d'un germe, il est en outre préférable de valider la sélection par dosage chromatographique.

Ainsi, selon l'invention il est maintenant possible d'obtenir en quantité industrielle, un nouvelle forme purifiée de streptogramine dont le taux d'impuretés, la définition et la constance de la composition est conforme aux exigences des législations sur l'enregistrement et qui possède de surcroît une activité in vivo et une biodisponibilité améliorées ainsi qu'une moindre toxicité. La nouvelle association pourra ainsi pallier à l'absence de traitement par un antibactérien de cette classe dans de nombreux pays.

La nouvelle association d'une composante du groupe B des streptogramines de formule générale (I) et d'une composante du groupe A des streptogramines de formule générale (II) présente une activité in vivo particulièrement intéressante notamment sur les germes à Gram-positif. In vivo, chez la souris elle s'est montrée active sur Staphylococcus aureus IP 8203 à des doses de 30 à 50 mg/kg par voie orale.

A titre d'exemple la DC₅₀ par voie orale de plusieurs associations des composantes de formule générale (I) et (II), dans l'infection expérimentale de la souris à Staphylococcus aureus IP 8203, sont données ci-après.

Dans le tableau I ci-après, les associations étudiées sont préparées par coprécipitation dans l'hexane, à partir d'une solution des composantes de formule générale (I) et (II) dans la méthylisobutylcétone ou dans l'acétone :

**Tableau I**

| Association produit (I) / produit (II) : PI (exemple 1)/PIIB (exemple 18) | DC₅₀ (mg/kg) p.o. |
|---|---|
| 10/90 | 44 |
| 20/80 | 32 |
| 30/70 | 30 |
| 70/30 | 30 |
| 80/20 | 30 |
| 90/10 | 50 |

Dans le tableau II ci-après les associations illustrées sont des produits cocristallisés préparés comme décrits dans les exemples.

**Tableau II**

| Association produit (I) / produit (II) cocristallisés | DC₅₀ (mg/kg) p.o. |
|---|---|
| PI/PIIB (exemple 9) | 38 |
| PIA/PIIB (exemple 11) | 28 |
| PIB/PIIB (exemple 12) | 32 |
| PIC/PIIB (exemple 13) | 36 |

| Association produit (I) / produit (II) cocristallisés | DC₅₀ (mg/kg) p.o. |
|---|---|
| PID/PIIB (exemple 14) | 50 |
| Facteur S/PIIB (exemple 15) | 32 |
| Facteur S1/PIIB (exemple 16) | 50 |
| Facteur S/PIIF (exemple 17) | 50 |

Dans le tableau III ci-après l'association décrite est préparée sous forme d'un mélange physique des poudres.

**Tableau III**

| Association produit (I) / produit (II) | DC₅₀ (mg/kg) p.o. |
|---|---|
| PIA (exemple 1)/PIIB (exemple 18) 30/70 | 36 |
| PIA (exemple 1)/PIIB (exemple 18) 50/50 | 40 |
| Facteur S (exemple 5)/PIIB (exemple 18) 30/70 | 44 |

De plus la nouvelle association ne présente pas de toxicité : aucun signe de toxicité ne se manifeste chez la souris à la dose de 150 mg/kg par voie orale (2 administrations).

Selon l'invention, lorsque l'association cocristallisée est utilisée comme moyen de purification de la composante de formule générale (II), celle-ci peut être obtenue par extraction acide d'une solution du composé cocristallisé dans une cétone (méthylisobutylcétone par exemple), puis isolement par extraction de la composante du groupe A par précipitation à partir de la phase organique.

Les exemples suivants donnés à titre non limitatif illustrent la présente invention.
Dans les exemples qui suivent il est entendu que les titres sont donnés en % en poids.

### SEPARATION ET PURIFICATION DE COMPOSANTES DU GROUPE B :

### Exemple 1

30 kg de pristinamycine brute [pristinamycine IA (PIA) : 20,7 %, pristinamycine IB (PIB) : 3,9 %, pristinamycine IC (PIC) : 0,6 %, pristinamycine ID (PID) : 0,3 %, pristinamycine IIB (PIIB) : 8 %, pristinamycine IIA (PIIA): 45 %, pristinamycine IIF (PIIF) : < 0,5 % (non dosé), pristinamycine IIG (PIIG) : < 0,5 % (non dosé)] sont mis en suspension dans 210 litres d'acétate d'éthyle et agités pendant 15 heures à température ambiante. La suspension est filtrée et le filtrat acétate d'éthyle recueilli est extrait par 2 fois 20 litres d'acide sulfurique 1N puis 20 litres d'eau distillée. Les phases aqueuses réunies sont lavées par 6 fois 15 litres d'acétate d'éthyle, puis ajustées à pH 7 par addition de 30 litres d'une solution à 10 % de bicarbonate de sodium et extraites par 3 fois 30 litres de chlorure de méthylène. Les phases chlorométhylèniques sont réunies et lavées par 10 litres d'eau distillée. Le chlorure de méthylène est ensuite distillé et remplacé par 50 litres d'éthanol. Le mélange est alors traité au reflux par 0,8 kg de noir L3S pendant 30 minutes. Après filtration et lavage par 2 fois 5 litres d'éthanol, le mélange est refroidi jusqu'à 10°C en 15 heures. Après maintien une heure à 10°C, la suspension est filtrée et lavée par 3 fois 7 litres d'éthanol. Après séchage du solide à 40°C sous pression réduite, on obtient 5,7 kg de pristinamycine I purifiée (ci-après appelée PI).
Titre: 96,8 % (PIA : 81,1 %, PIB : 12 %, PIC : 2,6 %, PID : 1,1 %);
Rendement en PIA : 74 %.

1500 g de PI purifiée sont repris par 9 litres de dichloro-1,2 éthane puis additionnés de 1,5 équivalent d'anhydride succinique et de 0,015 équivalent de diméthylaminopyridine. La solution est maintenue 1 semaine à 20°C, puis introduite sur une colonne contenant 10 kg de silice (20-45 µm) [hauteur de la colonne : 1 m ; diamètre : 20 cm]. L'élution est effectuée par percolation d'un mélange dichloro-1,2 éthane/méthanol à un débit de 18 litres/heure pendant 6 heures; le pourcentage de méthanol (à 5 % de teneur en eau) est augmenté de 0 à 4 % au cours de la chromatographie. 47 fractions de 2,4 litres sont récupérées.

Les fractions 5 à 15 sont réunies, le dichloro-1,2 éthane est évaporé et remplacé par 5 litres d'éthanol. Après cristallisation, on obtient 365 g de PIA titrant 99,8 %.

### Exemple 2

Les fractions 36 à 39 de la chromatographie décrite à l'exemple 1 sont réunies, et le dichloro-1,2 éthane est distillé; 210 g de solide sont ainsi obtenus. 40 g de ce solide est repris par 8 litres d'eau auquel sont ajouté 8 cm³ d'acide chlorhydrique 10 N. Après 3 heures à 90°C, on neutralise la solution à pH 6,5 avec de l'hydrogéno-carbonate de sodium. La solution est extraite par 3 fois 1 litre d'acétate d'éthyle, et l'extrait lavé par 2 fois 0,2 litre d'eau. Après traitement au noir, l'acétate d'éthyle est évaporé, et remplacé par 600 cm³ d'éthanol. Après recristallisation, on obtient 20 g de PIB titrant 97 %.

### Exemple 3

Les fractions 22 à 26 de la chromatographie décrite à l'exemple 1 sont réunies, et le dichloro-1,2 éthane est distillé; 139 g de solide sont ainsi obtenus. Ce solide est repris par un minimum de dichloro-1,2 éthane, et introduit sur une colonne de silice. L'élution est effectuée par percolation d'un mélange dichloro-1,2 éthane/méthanol à un débit de 18 litres/heure pendant 6 heures; le pourcentage de méthanol (à 5 % de teneur en eau) est augmenté de 0 à 5 % au cours de la chromatographie. 48 fractions de 2,4 litres sont récupérées. Les fractions 38 à 43 sont évaporées et le solide repris par 300 cm³ d'éthanol. Après recristallisation, on obtient 22 g de PI à 40 % de PIC. Des chromatographies successives sur silice (20-45 µm) avec percolation par un éluant chlorure de méthylène/méthanol (98/2 en volumes) permettent d'obtenir 5 g d'un solide qui, après battage à la méthyl-isobutylcétone et recristallisation dans l'éthanol, titre 95 % en PIC.

### Exemple 4

1000 g de PI obtenus comme décrit précédemment à l'exemple 1, sont mis en solution dans le minimum de chloroforme et purifiés par fractions successives sur colonne de silice (20-45 µm). Après élution par du chloroforme contenant 2 à 5 % de méthanol, on obtient un produit qui est concentré à sec. Ce produit est ensuite purifié par 2 passages successifs sur colonne de résine Diaion® percolée avec un mélange acétonitrile/eau (60/40 en volumes). Les fractions sont contrôlées par chromatographie. Les fractions contenant la PID sont réunies et concentrées à sec. On obtient ainsi, environ 3 g de produit titrant 60 % en PID. Une purification complémentaire est effectuée par chromatographie à contre-courant en utilisant le mélange de solvants méthylisobutylcétone/acétone/acide formique (40/2/40 en volumes). La concentration à sec des fractions contenant la PID permet d'obtenir 1 g d'un solide titrant 95 % de PID.

### Exemple 5

400 g de Staphylomycine® (sous forme de comprimés - composition initiale: virginiamycine S1 (S1) : 3,4 %, virginiamycine S4 (S4): 0,9 %) sont introduits dans 4 litres d'eau.

Les comprimés sont désagrégés par agitation pendant 15 minutes à 20°C. On ajoute 1 litre de chlorure de méthylène et on poursuit l'agitation pendant 1 heure. La phase chlorométhylènique est ensuite décantée et filtrée, puis versée en 30 minutes dans un volume de 5 litres d'hexane agité. Après 1 heures d'agitation, on filtre la suspension et on recueille un solide qui est lavé par 3 fois 25O cm³ d'hexane. Après séchage, on récupère 52 g de solide qui est mis en suspension dans 370 cm³ d'acétate d'éthyle. 2 battages successifs à 20°C et d'une durée de 18 heures sont réalisés. Le filtrat correspondant à chaque battage est porté à sec, puis dissous dans 850 cm³ de méthanol au reflux. Après descente progressive de la température jusqu'à -2O°C en 16 heures, on recueille par filtration un solide qui est lavé par une petite quantité de méthanol. Après séchage du solide à 35°C sous pression réduite, on obtient 9 g de facteur S (virginiamycine S).
Titre: 96 % (S1 : 75,4 %, S4 : 20,6 %).
Rendement en facteur S1 (virginyamycine S1) : 50 %.

### Exemple 6

1 g de facteur S obtenu comme décrit précédemment à l'exemple 5, mis en solution dans l'acétonitrile à raison de 125 mg/cm³, est purifié en 4 opérations par chromatographie sur colonne de Nucléosil5C8® (hauteur 25 cm, diamètre extérieur 2,54 cm) en injectant un volume de 2 cm³ et en éluant par un mélange eau/acétonitrile 60/40 en volumes, sous un débit de 7,5 cm³/minute. On recueille à chaque fois un volume de 120 cm³ contenant le facteur S1, soit 480 cm³ en tout. La chromatographie est répétée 4 fois afin de traiter la totalité des 1 g de facteur S. On recueille ainsi un volume d'environ 500 cm³ contenant le facteur S1. L'acétonitrile est éliminé au moyen d'un évaporateur rotatif. La phase aqueuse est extraite par 3 fois 50 cm³ de dichlorométhane. Les phases chlorométhyléniques sont rassemblées, lavées par 50 cm³ d'eau distillée, séchées sur sulfate de sodium et filtrées. Le dichlorométhane est éliminé au moyen d'un évaporateur rotatif, sous pression réduite (5 mm de mercure). On obtient ainsi 0,67 g de facteur S1 titrant 99,6 %.

### PREPARATION DE COMPOSANTES BRUTES DU GROUPE A :

### Exemple 7

500 g de pristinamycine brute [pristinamycine IA (PIA) : 20,7 %, pristinamycine IB (PIB) : 3,9 %, pristinamycine IC (PIC) : 0,6 %, pristinamycine ID (PID) : 0,3 %, pristinamycine IIB (PIIB) : 8 %, pristinamycine IIA (PIIA): 45 %] sont mis en solution dans 50 litres de méthylisobutylcétone. Cette solution est extraite 5 fois par une phase aqueuse composée de 2,5 litres d'eau et 2,5 litres d'acide sulfurique 1 N, puis lavée par 3 fois 10 litres d'eau. La méthylisobutylcétone est ensuite traitée par 7,5 litres d'une solution aqueuse d'hydrogénocarbonate de sodium à 35 g/litre, puis lavée par 5 litres d'eau. A chaque fois, la phase aqueuse est mélangée avec la phase organique, décantée et séparée.

La phase organique obtenue est mise en contact avec 750 g d'alumine, filtrée, concentrée jusqu'à un volume de 4 litres environ et reprise par 5 volumes d'hexane. Le précipité obtenu est filtré et séché. On obtient 300 g de produit que l'on met en suspension dans 1 litre d'isopropanol. Après agitation à 55°C pendant 45 minutes, on filtre à 4°C. Les eaux-mères de filtration sont concentrées à sec, reprises par 500 cm³ de méthylisobutylcétone sur lesquels on verse 5 volumes d'hexane. Le précipité est filtré, lavé à l'hexane et séché à 40°C sous pression réduite. On obtient 69 g de PIIB brute contenant 36 % de PIIB, 6 % de PIIA et ne contenant plus de PIA.

### Exemple 8

60 g de PIIB brute obtenue comme précédemment à l'exemple 7 sont purifiés en plusieurs opérations, par chromatographie sur colonne de Nucléosil5C8® (diamètre de la colonne 5 cm, hauteur 30 cm) percolée par un éluant eau/acétonitrile 60/40. On obtient ainsi 250 mg de pristinamycine IIF (PIIF).

### PREPARATION D'UN PRODUIT COCRISTALLISE :

Dans les exemples qui suivent, il a été démontré que le spectre de diffraction X du produit cocristallisé est différent du spectre de la composante du groupe B cristallisée seule dans le même solvant, lorsqu'elle existe.

### Exemple 9

La PIIB brute obtenue précédemment à l'exemple 7 est mise en solution dans 190 cm³ d'acétone. On ajoute 33 g de PI purifiée (PIA : 81,1 %, PIB : 12 %, PIC : 2,6 %, PID : 1,1 %). Après 17 heures d'agitation à 20°C, on obtient une suspension qui est filtrée à 4°C. Le produit est lavé et séché. Après une recristallisation à 100 g/l dans l'acétone, on obtient 10 g de cristaux blancs dont le titre en PIIB+PIIF+PIIG est 55 % et le titre en PIA+PIB+PIC+PID est 43 %.

### Exemple 10

250 mg de PIIB purifiée, obtenue comme décrit ci-après à l'exemple 18, sont mis en solution dans 17 cm³ d'acétate d'éthyle. On ajoute 300 mg de PI purifiée (PIA : 81,1 %, PIB : 12 %, PIC : 2,6 %, PID : 1,1 %). Après 20 heures d'agitation à 20°C, filtration, lavage et séchage, on obtient 125 mg de de cristaux blancs.
Titre en PIIB+PIIF+PIIG : 56 % ; dont PIIB 54 %.
Titre en PIA+PIB+PIC+PID : 43 %.

### Exemple 11

560 mg de PIIB pure obtenus comme décrit ci-après à l'exemple 18, sont mis en solution dans 5 cm³ d'acétone. On ajoute 480 mg de PIA (titre 99,8 %). On agite 20 heures à 20°C et filtre la suspension. Après lavage par 1 cm³ d'acétone et séchage pendant 30 heures à 40°C sous pression réduite (<1 kPa), on obtient 590 mg de cristaux blancs.
Titre en PIIB+PIIF+PIIG : 56 % ; dont PIIB 54 %.
Titre en PIA: 43 %.

Les eaux mères de la cristallisation précédente sont reprises et additionnées d'une nouvelle charge de 560 mg de PIIB. Le rapport massique PIIB/PIA est alors voisin de 4. Après 20 heures d'agitation, filtration, lavage et séchage, on obtient 195 mg de cristaux de pureté et de composition identiques à celles des cristaux issus du 1er jet.

### Exemple 12

En opérant comme décrit ci-dessus à l'exemple 11 mais en remplaçant la PIA par 480 mg de PIB (titre 97 %) on obtient 820 mg de cristaux blancs dont le titre en PIIB+PIIF+PIIG est 56 % (dont PIIB 54 %) et le titre en PIB est 43 %.

### Exemple 13

En opérant comme décrit ci-dessus à l'exemple 11 mais en engageant 680 mg de PIIB et 580 mg de PIC (titre 95 %) dans 4 cm³ d'acétone on obtient 315 mg de cristaux blancs dont le titre en PIIB+PIIF+PIIG est 57 % (dont PIIB 55 %) et le titre en PI est 42 % (dont 37 % de PIC).

### Exemple 14

En opérant comme décrit ci-dessus à l'exemple 11 mais en remplaçant la PIA par 480 mg de PID (titre 95 %) on obtient 475 mg de cristaux blancs dont le titre en PIIB+PIIF+PIIG est 55 % (dont PIIB 53 %) et le titre en PID est 39 %.

### Exemple 15

En opérant comme décrit ci-dessus à l'exemple 11 mais en engageant 450 mg de PIIB et 380 mg de facteur S (S1 : 75,4 %, S4 : 20,6 %) dans 4 cm³ d'acétone on obtient 550 mg de cristaux blancs dont le titre en PIIB+PIIF+PIIG est 58 % (dont PIIB 56 %) et le titre en facteur S est 41 % (dont 37 % de S1).

### Exemple 16

En opérant comme décrit ci-dessus à l'exemple 11 mais en remplaçant la PIA par 480 mg de facteur S1, on obtient 750 mg de cristaux blancs dont le titre en PIIB+PIIF+PIIG est 58 % (dont PIIB 54 %) et le titre en facteur S1 est 41 %.

### Exemple 17

En opérant comme décrit ci-dessus à l'exemple 11 mais en engageant 224 mg de PIIF et 192 mg de Facteur S dans 2 cm³ d'acétone, on obtient 220 mg de cristaux blancs dont le titre en PIIF est 55 % et le titre en facteur S est 39 % (dont facteur S1 : 31 %, facteur S4 : 5 %).

### Diagrammes de diffraction X des produits des exemples 9 à 17 :

Dans le tableau IV ci-après sont données les intensités relatives des raies principales. Les diagrammes de diffraction X sont effectués sur diffractomètre Phillips PW1700 à anticathode de cobalt. La référence 100 est donnée pour la raie à 15,8 Å. Les valeurs relatives sont estimées par la mesure de la hauteur de la raie, fond continu déduit.

**Tableau IV**

| distance interréticulaire | Produit de l'exemple | | | | | | | |
|---|---|---|---|---|---|---|---|---|
| (Å) | Ex. 9 | Ex. 11 | Ex. 12 | Ex. 13 | Ex. 14 | Ex. 15 | Ex. 16 | Ex. 17 |
| 15,8 | 100 | 100 | 100 | 100 | 100 | 100 | 100 | 100 |
| 11,8 | 40 | 34 | 35 | 32 | 43 | 28 | 36 | 21 |
| 10,3 | 69 | 52 | 63 | 65 | 50 | 70 | 80 | 87 |
| 9,7 | 38 | 45 | 47 | 44 | 43 | 40 | 49 | 45 |
| 6,4 | 44 | 41 | 51 | 41 | 37 | 40 | 51 | 39 |
| 6,1 | 38 | 41 | 40 | 38 | 43 | 30 | 40 | 35 |
| 5,9 | 75 | 64 | 70 | 63 | 67 | 74 | 89 | 71 |
| 5,8 | 38 | 39 | 47 | 49 | 50 | 44 | 54 | 35 |
| 5,2 | 92 | 75 | 79 | 71 | 70 | 70 | 91 | 81 |
| 5,0 | 67 | 59 | 60 | 60 | 57 | 54 | 69 | 52 |
| 4,7 | 50 | 43 | 53 | 49 | 47 | 50 | 40 | 42 |

Les diagrammes de diffraction X sont similaires quel que soit le produit cocristallisé (les distances interréticulaires des raies principales ne sont pas significativement différentes).

### PURIFICATION D'UNE COMPOSANTE DU GROUPE A :

### Exemple 18

9,3 g du produit obtenu à l'exemple 9 sont dissous dans 490 cm³ de méthylisobutylcétone. Cette solution est extraite deux fois par 370 cm³ d'une solution aqueuse d'acide sulfurique 0,5 N puis lavée par deux fois 150 cm³ d'eau. La phase organique est ensuite concentrée jusqu'à un volume de 80 cm³ environ que l'on verse sur 5 volumes d'hexane. Le précipité obtenu est lavé, filtré et séché. Pour éliminer la méthylisobutylcétone, il est ensuite repris à 100 g/l dans l'acétone, versé sur 10 volumes d'hexane, lavé et séché. On obtient 3,5 g d'un produit contenant la PIIB purifiée et ne contenant plus de PI.
Titre PIIB+PIIF+PIIG: environ 95 % dont 92 % de PIIB.

La présente invention concerne également les compositions pharmaceutiques utilisables en médecine humaine ou vétérinaire qui contiennent comme produit actif la nouvelle association purifiée de streptogramine comprenant au moins une composante du groupe B des streptogramines associée à la composante du groupe A de formule générale (II), à l'état pur ou en présence d'un ou plusieurs diluants ou adjuvants compatibles et pharmaceutiquement acceptables. Ces compositions peuvent être utilisées par voie orale ou topique. Elles peuvent contenir les associations selon l'invention à l'état de mélange physique des poudres, de coprécipitat ou de cocristallisat

Comme compositions pour administration orale, peuvent être utilisés des comprimés, des gélules, des pilules, des poudres des lyophilisats ou des granulés. Dans ces compositions, le produit actif selon l'invention peut être mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Les compositions pour administration topique peuvent être par exemple des crèmes, des pommades, ou des lotions.

En thérapeutique humaine ou vétérinaire, les compositions selon l'invention sont particulièrement utiles dans le traitement des infections d'origine bactérienne notamment les infections graves à cocci Gram-positif : infections à staphylocoques (notamment les infections à staphylocoque résistant à la méthicilline), infections à streptocoques (notamment sur les pneumocoques résistants à la pénicilline et aux macrolides) ; ils sont aussi particulièrement utiles dans le traitement des infections à Hemophilus, Moraxella catarrhalis, Neisseria gonorrhoeae, Chlamydia trachomatis, Mycoplasma hominis, Mycoplasma pneumoniae, Ureaplasma urealyticum.

Les compositions selon l'invention peuvent être employées notamment dans le traitement des infections respiratoires hautes et basses (par exemple traitement des infections pulmonaires), dans le traitement des infections cutanées, dans le traitement à long terme des infections osseuses ou articulaires, dans le traitement ou la prophylaxie de l'endocardite en chirurgie dentaire et urinaire, dans le traitement des maladies sexuellement transmisibles, ainsi que dans le traitement des infections opportunistes bactériennes et parasitaires survenant dans le SIDA et en prophylaxie du risque staphylococcique chez l'immunodéprimé.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 0,4 et 3,5 g de produit actif en 2 ou 3 prises par jour, par voie orale pour un adulte.

Les exemples suivants, donnés à titre non limitatif, illustrent des compositions selon l'invention :

### Exemple A

On prépare selon les techniques habituelles des gélules opaques dosées à 250 mg de l'association PIB/PIIB cocristallisée.

### Exemple B

On prépare selon les techniques habituelles des gélules opaques dosées à 250 mg de l'association Facteur S/PIIB cocristallisé.

### Exemple C

On prépare selon les techniques habituelles des comprimés dosés à 384 mg de produit actif, ayant la composition suivante :

| | |
|---|---|
| - PIB/PIIB (45%/55%) | 384 mg |
| - Hydroxypropylméthylcellulose | 25 mg |
| - Stéarate de magnésium | 35 mg |
| - Silice colloïdale | 14 mg |
| - Amidon qsp | 700 mg |

### Exemple D

On prépare selon les techniques habituelles des comprimés dosés à 384 mg de produit actif, ayant la composition suivante :

| | |
|---|---|
| - Facteur S/PIIB (45%/55%) | 384 mg |
| - Hydroxypropylméthylcellulose | 25 mg |
| - Stéarate de magnésium | 35 mg |
| - Silice colloïdale | 14 mg |
| - Amidon qsp | 700 mg |

## Revendications

1. Une forme purifiée de streptogramines caractérisée en ce qu'elle est constituée de l'association d'une ou plusieurs composantes du groupe B des streptogramines, de formule générale : 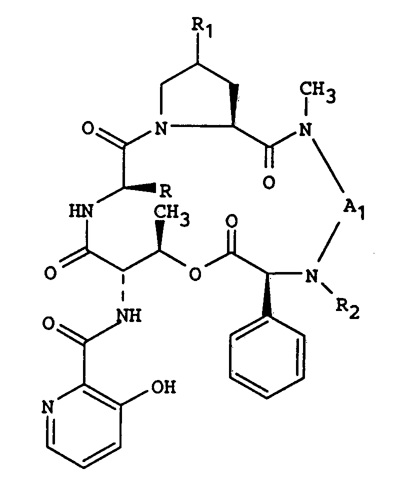
dans laquelle A₁ est un radical de formule générale : 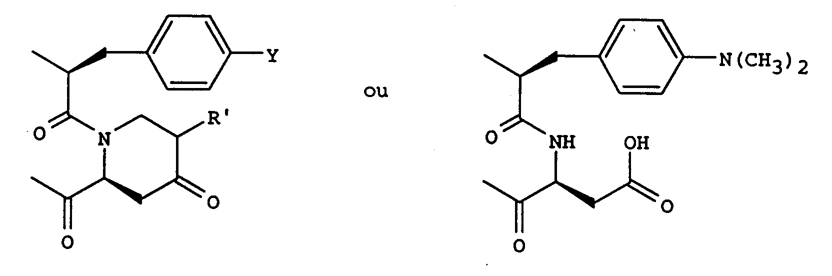
pour lequel R' est un atome d'hydrogène ou un radical hydroxy et Y est un atome d'hydrogène, un radical méthylamino ou un radical diméthylamino,
R est un radical éthyle ou lorsque R' représente un atome d'hydrogène
R peut également représenter méthyle, et
R₁ et R₂ représentent un atome d'hydrogène ou bien,
A₁ est un radical de formule : 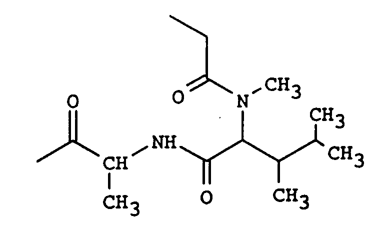
R est un radical isobutyle, et
R₁ est un radical hydroxy et R₂ est un radical méthyle,
et d'une ou plusieurs composantes minoritaires du groupe A des streptogramines, de formule générale : 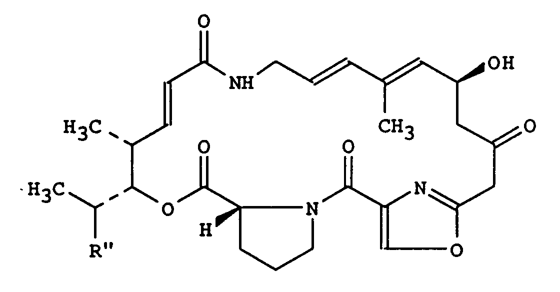
dans laquelle R'' est un atome d'hydrogène ou un radical méthyle ou éthyle, à l'état de cocristallisat, de coprécipitat ou de mélange physique des poudres.

2. Une forme purifiée de streptogramines selon la revendication 1 caractérisée en ce qu'elle contient moins de 6 % d'impuretés.

3. Une forme purifiée de streptogramines selon l'une des revendications 1 ou 2, constituée d'une association dans des proportions de 10/90 à 90/10 (en poids), respectivement d'une ou plusieurs composantes du groupe B des streptogramines et d'une ou plusieurs composantes minoritaires du groupe A des streptogramines telles que définies dans la revendication 1, à l'état de coprécipitat ou de mélange physique des poudres.

4. Une forme purifiée de streptogramines selon l'une des revendications 1 à 3, constituée d'une association de 20/80 à 80/20 (en poids) respectivement d'une ou plusieurs composantes du groupe B des streptogramines et d'une ou plusieurs composantes minoritaires du groupe A des streptogramines telles que définies dans la revendication 1, à l'état de coprécipitat ou de mélange physique des poudres.

5. Une forme purifiée de streptogramines selon la revendication 1 ou 2, caractérisée en ce qu'il s'agit d'un composé cocristallisé d'une ou plusieurs composantes du groupe B des streptogramines et d'une ou plusieurs composantes minoritaires du groupe A des streptogrammes telles que définies dans la revendication 1, dans un rapport molaire de l'ordre de 1/2.

6. Utilisation d'un composé cocristallisé selon la revendication 5 pour la préparation d'une forme purifiée de streptogramines selon l'une des revendications 1 à 4.

7. Utilisation d'un composé cocristallisé selon la revendication 5 pour la purification d'une composante minoritaire du groupe A des streptogramines telle que définie dans la revendication 1.

8. Un procédé de préparation d'une forme purifiée de streptogramines selon l'une des revendications 1 à 5, caractérisé en ce que l'on cocristallise la(les) composante(s) du groupe A des streptogramines, avec la(les) composantes du groupe B des streptogramines telles que définies dans la revendication 1, puis le cas échéant associe le composé cocristallisé obtenu avec la (ou les) composante(s) convenable(s) du groupe A ou B pour obtenir une association dans les proportions souhaitées.

9. Un procédé de purification d'une composante minoritaire du groupe A des streptogramines définie dans la revendication 1, caractérisé en ce que l'on prépare un composé cocristallisé de cette composante avec une ou plusieurs composantes du groupe B des streptogramines, puis élimine la (les) composantes du groupe B par traitement en milieu acide.

10. Une composante purifiée du groupe A des streptogramines telle que définie dans la revendication 1, lorsqu'elle est obtenue par l'intermédiaire d'un composé cocristallisé selon la revendication 5.

11. Une composante du groupe A des streptogramines de formule générale : 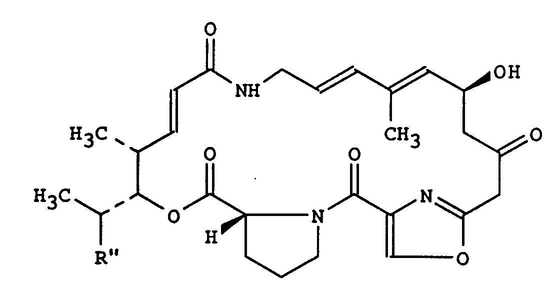 dans laquelle R'' est un atome d'hydrogène ou un radical éthyle.

12. Utilisatibn d'une ou plusieurs composantes minoritaires du groupe A des streptogramines telles que définies dans la revendication 1, pour la préparation de cocristallisats avec une ou plusieurs composantes du groupe B des streptogramines ou leurs dérivés.

13. Composés cocristallisés constitués d'une ou plusieurs composantes du groupe B des streptogramines ou de leurs dérivés, et d'une ou plusieurs composantes minoritaires du groupe A des streptogramines définies dans la revendication 1.

14. Composition pharmaceutique caractérisée en ce qu'elle contient une forme purifiée de streptogramines selon l'une des revendications 1 à 5, à l'état pur ou en présence de tout diluant ou adjuvant compatible et pharmaceutiquement acceptable.

## Claims

1. A purified form of streptogramins, characterized in that it consists of the combination of one or more group B components of streptogramins, of general formula: 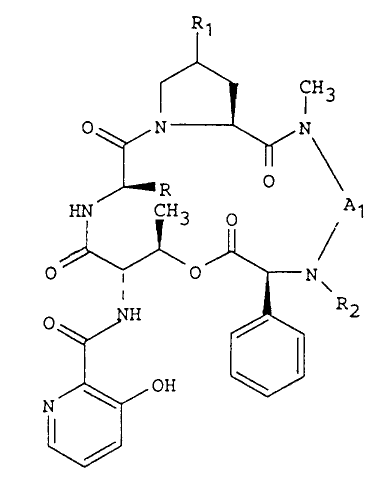
in which A₁ is a radical of general formula: 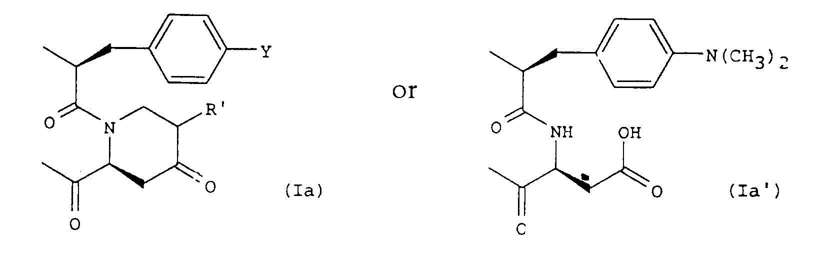
for which R' is a hydrogen atom or a hydroxyl radical and Y is a hydrogen atom, a methylamino radical or a dimethylamino radical,
R is an ethyl radical or, when R' represents a hydrogen atom, R can also represent a methyl radical, and
R₁ and R₂ represent a hydrogen atom, or alternatively
A₁ is a radical of formula: 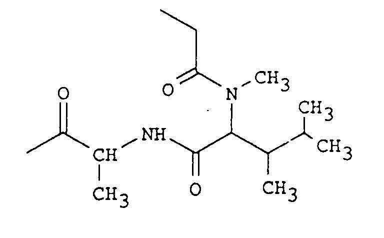
R is an isobutyl radical, and
R₁ is a hydroxyl radical and R₂ is a methyl radical,
and one more group A minority components of streptogramins, of general formula: 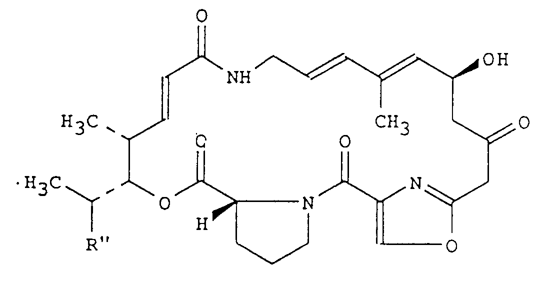
in which R'' is a hydrogen atom or a methyl or ethyl radical, in the state of a cocrystallizate, of a coprecipitate or of a physical mixture of the powders.

2. A purified form of streptogramins according to claim 1, characterized in that it contains less than 6 % of impurities.

3. A purified form of streptogramins according to one of claims 1 and 2, consisting of a combination in proportions of 10:90 to 90:10 (by weight), respectively, of one or more group B components of streptogramins and one or more group A minority components of streptogramins as are defined in claim 1, in the state of a coprecipitate or of a physical mixture of the powders.

4. A purifed form of streptogramins according to one of claims 1 to 3, consisting of a combination of 20:80 to 80:20 (by weight), respectively, of one or more group B components of streptogramins and one or more group A minority components of streptogramins as are defined in claim 1, in the state of a coprecipitate or of a physical mixture of the powders.

5. A purified form of streptogramins according to claim 1 or 2, characterized in that it is a cocrystallized compound of one or more group B components of streptogramins and one or more group A minority components of streptogramins as are defined in claim 1, in a mole ratio of the order of 1:2.

6. Use of a cocrystallized compound according to claim 5, for the preparation of a purified form of streptogramins according to one of claims 1 to 4.

7. Use of a cocrystallized comound according to claim 5, for the purification of a group A minority component of streptogramins as is defined in claim 1.

8. A process for preparing a purified form of streptogramins according to one of claims 1 to 5, characterized in that the group A component(s) of streptogramins is/are cocrystallized with the group B component(s) of streptogramins as are defined in claim 1, and the cocrystallized compound obtained is then, if need be, combined with the appropriate group A or B component(s) to obtain a combination in the desired proportions.

9. A process for purifying a group A minority component of streptogramins which is defined in claim 1, characterized in that a cocrystallized compound of this component with one or more group B components of streptogramins is prepared, and the group B component(s) is/are then removed by treatment in an acid medium.

10. A purified group A component of streptogramins as is defined in claim 1, when it is obtained via a cocrystallized compound according to claim 5.

11. A group A component of streptogramins of general formula: 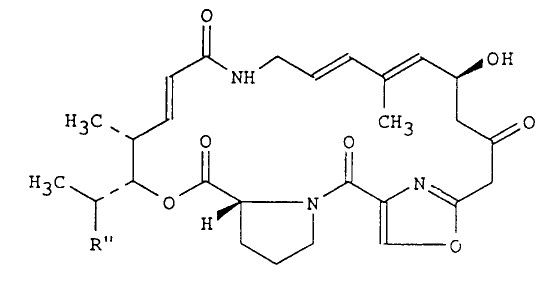 in which R'' is a hydrogen atom or an ethyl radical.

12. Use of one or more group A minority components of streptogramins as are defined in claim 1, for the preparation of cocrystallizates with one or more group B components of streptogramins or their derivatives.

13. Cocrystallized compounds consisting of one or more group B components of streptogramins or their derivatives, and one or more group A minority components of streptogramins defined in claim 1.

14. Pharmaceutical composition, characterized in that it contains a purified form of streptogramins according to one of claims 1 to 5, in the pure state or in the presence of any compatible and pharmaceutically acceptable diluent or adjuvant.

## Patentansprüche

1. Streptogramine in gereinigter Form, dadurch gekennzeichnet, daß sie aus der Assoziation
eines oder mehrerer Vertreter der Gruppe B der Streptogramine der folgenden allgemeinen Formel: 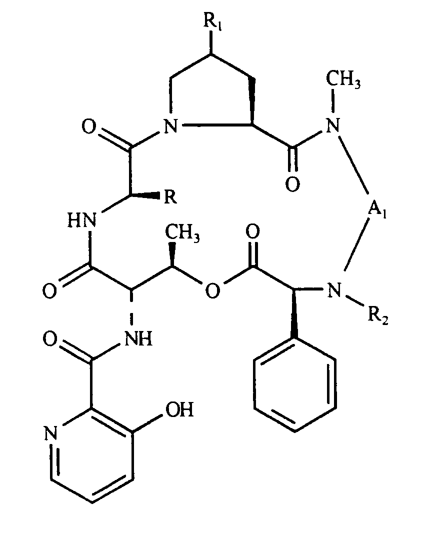 in der:
• A₁ entweder ein Rest der folgenden allgemeinen Formel ist: 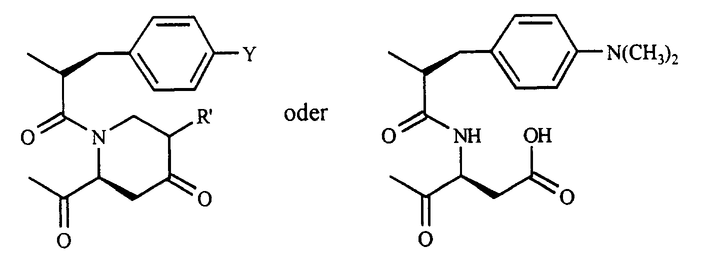 wobei in diesem Fall:
- R' ein Wasserstoffatom oder ein Hydroxylrest und Y ein Wasserstoffatom, ein Methylaminorest oder ein Dimethylaminorest ist,
- R ein Ethylrest ist oder, wenn R' ein Wasserstoffatom darstellt, auch einen Methylrest darstellen kann
und
- R₁ und R₂ jeweils ein Wasserstoffatom darstellen,
oder
• A₁ ein Rest der folgenden Formel ist: 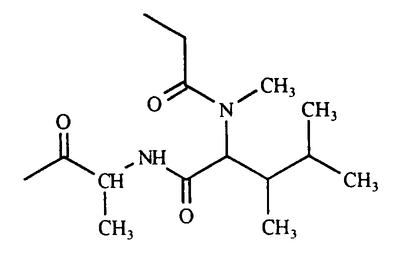 wobei in diesem Fall:
- R ein Isobutylrest ist,
- R₁ ein Hydroxylrest und R₂ ein Methylrest ist
und
eines oder mehrerer minoritärer Vertreter der Gruppe A der Streptogramine der folgenden allgemeinen Formel: 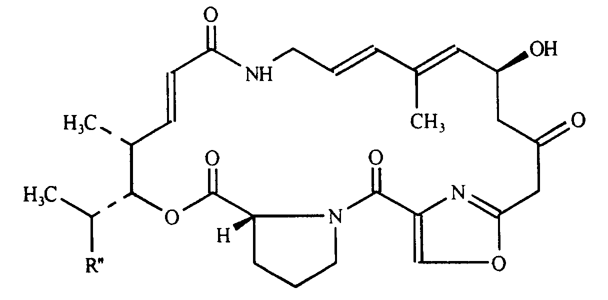 in der R'' ein Wasserstoffatom oder ein Methyl- oder Ethylrest ist,
in Form eines Cokristallisats, eines Copräzipitats oder eines physikalischen Gemischs der pulverförmigen Feststoffe bestehen.

2. Streptogramine in gereinigter Form nach Anspruch 1, dadurch gekennzeichnet, daß sie weniger als 6% Verunreinigungen enthalten.

3. Streptogramine in gereinigter Form nach einem der Ansprüche 1 und 2, bestehend aus einer Assoziation eines oder mehrerer Vertreter der Gruppe B der Streptogramine wie in Anspruch 1 definiert und eines oder mehrerer minoritärer Vertreter der Gruppe A der Streptogramine wie in Anspruch 1 definiert in einem Gewichtsverhältnis von 10/90 bis 90/10 in Form eines Copräzipitats oder eines physikalischen Gemischs der pulverförmigen Feststoffe.

4. Streptogramine in gereinigter Form nach einem der Ansprüche 1 bis 3, bestehend aus einer Assoziation eines oder mehrerer Vertreter der Gruppe B der Streptogramine wie in Anspruch 1 definiert und eines oder mehrerer minoritärer Vertreter der Gruppe A der Streptogramine wie in Anspruch 1 definiert in einem Gewichtsverhältnis von 20/80 bis 80/20 in Form eines Copräzipitats oder eines physikalischen Gemischs der pulverförmigen Feststoffe.

5. Streptogramine in gereinigter Form nach Anspruch 1 oder 2, dadurch gekennzeichnet, daß es sich hierbei um eine cokristallisierte Verbindung eines oder mehrerer Vertreter der Gruppe B der Streptogramine wie in Anspruch 1 definiert und eines oder mehrerer minoritärer Vertreter der Gruppe A der Streptogramine wie in Anspruch 1 definiert in einem Molverhältnis in der Größenordnung von 1/2 handelt.

6. Verwendung einer cokristallisierten Verbindung nach Anspruch 5 zur Herstellung von Streptograminen in gereinigter Form nach einem der Ansprüche 1 bis 4.

7. Verwendung einer cokristallisierten Verbindung nach Anspruch 5 zur Reinigung eines minoritären Vertreters der Gruppe A der Streptogramine wie in Anspruch 1 definiert.

8. Verfahren zur Herstellung von Streptograminen in gereinigter Form nach einem der Ansprüche 1 bis 5, dadurch gekennzeichnet, daß der beziehungsweise die Vertreter der Gruppe A der Streptogramine wie in Anspruch 1 definiert mit dem beziehungsweise den Vertretern der Gruppe B der Streptogramine wie in Anspruch 1 definiert cokristallisiert werden und die erhaltene cokristalliserte Verbindung erforderlichenfalls anschließend mit dem beziehungsweise den geeigneten Vertretern der Gruppe A oder B assoziiert werden, um eine Assoziation im gewünschten Mengenverhältnis zu erhalten.

9. Verfahren zur Reinigung eines minoritären Vertreters der Gruppe A der Streptogramine wie in Anspruch 1 definiert, dadurch gekennzeichnet, daß eine cokristallisierte Verbindung dieses Bestandteils mit einem oder mehreren Vertretern der Gruppe B der Streptogramine hergestellt wird und anschließend der beziehungsweise die Streptogramine der Gruppe B durch eine Behandlung in saurem Milieu entfernt werden.

10. Vertreter der Gruppe A der Streptogramine in gereinigter Form wie in Anspruch 1 definiert, sofern dieser über eine cokristallisierte Verbindung nach Anspruch 5 als Zwischenprodukt erhalten wird.

11. Vertreter der Gruppe A der Streptogramine der folgenden allgemeinen Formel: 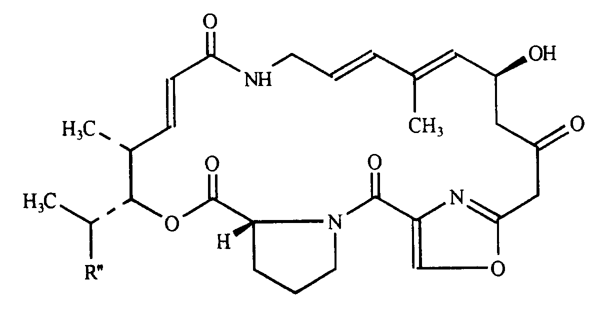
in der R'' ein Wasserstoffatom oder ein Ethylrest ist.

12. Verwendung eines oder mehrerer minoritärer Vertreter der Gruppe A der Streptogramine wie in Anspruch 1 definiert zur Herstellung von Cokristallisaten mit einem oder mehreren Vertretern der Gruppe B der Streptogramine oder deren Derivaten.

13. Cokristallisierte Verbindungen, bestehend aus einem oder mehreren Vertretern der Gruppe B der Streptogramine wie in Anspruch 1 definiert oder deren Derivaten und einem oder mehreren minoritären Vertretern der Gruppe A der Streptogramine wie in Anspruch 1 definiert.

14. Pharmazeutische Zusammensetzung, dadurch gekennzeichnet, daß sie Streptogramine in gereinigter Form nach einem der Ansprüche 1 bis 5 in reiner Form oder in Gegenwart eines beliebigen geeigneten und pharmazeutisch akzeptablen Verdünnungsmittels oder Zusatzstoffs enthält.
